# EUROPÄISCHE PATENTANMELDUNG

(11) **EP 2 336 767 A1**
(43) Veröffentlichungstag der Anmeldung: **22.06.2011**
(21) Anmeldenummer: 09015600.1
(22) Anmeldetag: 17.12.2009
(51) Int. Cl.: G01N 33/50, G01N 33/552, G01N 33/554

(54) **Mittel zum Nachweis biochemischer Aktivität von Membrankörpern**

(71) Anmelder: Bayer Technology Services GmbH, 51368 Leverkusen (DE)
(72) Erfinder: Habig, Michael, 50667 Köln (DE); Methfessel, Christoph, Dr., 42327 Wuppertal (DE)

(57) **Zusammenfassung**

Die Erfindung betrifft eine Messanordnung zum Nachweis von biochemischer Aktivität, wobei die nachzuweisende biochemische Aktivität innerhalb eines oder mehrerer Membrankörper und / oder die Membranaktivität der Membrankörper durch Fusion ihrer Membranen mit mindestens einer Lipidmembran in einem oder mehreren Membran-Konstrukten zur Untersuchung zugänglich gemacht wird, wobei das Membran-Konstrukt durch Inkontaktbringen einer Lipidmembran mit mindestens einem Membrankörper und Fusion der Membranen mit Hilfe Membranfusion induzierender Stoffe herstellbar ist und mindestens einen zu untersuchenden, biochemisch aktiven Stoff enthält Die beschriebene Messanordnung kann zum Screening bzw. High Throughput Screening von Wirkstoffe eingesetzt werden.

## Beschreibung

Die Erfindung betrifft eine Messanordnung zum Nachweis von biochemischer und / oder elcktrischer Aktivität, wobei die nachzuweisende biochemische und / oder elektrische Aktivität innerhalb eines Membrankörpers und / oder die Membranaktivität eines Membrankörpers durch Fusion seiner Membran mit mindestens einer Lipidmembran in einem Membran-Konstrukt zur Untersuchung zugänglich gemacht wird. Die erzeugten Membran-Konstrukte sind elektrophysiologisch oder biochemisch adressierbar. Weitere Gegenstände der vorliegenden Erfindung sind ein Verfahren zur Herstellung der erfindungsgemäßen Messanordnung und die Verwendung der erfindungsgemäßen Messanordnung in einem High Throughput Screening.

### Stand der Technik

Die Suche nach neuen Wirkstoffen, die an membranständigen oder membranumschlossenen Molekülen gebunden werden und ihre Funktion beeinflussen, ist eine herausragende Aufgabe der pharmaceutischen Forschung. Dazu existieren viele Messmethoden, die aber alle unterschiedliche Beschränkungen aufweisen und nicht die Genauigkeit, die Vielseitigkeit oder den hohen Durchsatz aufweisen, der zur optimalen Lösung der Aufgabe erforderlich wäre.

Erstrebenswert sind Verfahren, bei denen beliebige Membrankörper, die ein zu untersuchendes Target-Molekül enthalten, in einem standardisierten und möglichst automatisierten Verfahren für eine biochemische oder elektrische Messung zugänglich gemacht werden.

### Assays an künstlichen Lipidmembranen

Es ist bekannt, dass Ionenkanälen, Rezeptoren und andere Targetmoleküle in künstliche Lipidmembranen eingebaut werden, und dort durch geeignete Experimente funktionell untersucht werden können [Hanke, W. (1985) Reconstitution of Ion Channels. CRC Critical Reviews Biochemistry 19, 1-44]. Interessant sind besonders jene Verfahren, die eine künstliche Lipidmembran durch ein geeignetes Substrat in der Weise stabilisieren, dass sie mechanisch fester und länger haltbar werden sowie reproduzierbar erzeugt werden können [Sackmann E. and Tanaka M. (2000) Supported Membranes on soft polymer cushions: fabrication, characterization and applications TIBTECH 18, 58-64]. Als Substrate finden hier zum Beispiel Silicagele Verwendung, die ggf. zur Verbesserung der Stabilität und Fluidität der Membran mit einer polymeren Zwischenschicht versehen worden sind [Loidl-Stahlhofen et al. (2001) Solid-Supported Biomolecules on Modified Silica Surfaces - A Tool for Fast Physicochemical Characterization and High-Throughput Screening. Advanced Materials 13, 1829-1834]. Auch können Bilayer auf dem Substrat mit gecigneten Kettenmolekülen stabilisiert werden ('tethered bilayers') [Raguse et al. (1998). Tethered Lipid Bilayer Membrdnes: Formation, and lonic Reservoir Characterization. Langmuir 14, 648-659].

Im Stand der Technik sind Verfahren beschrieben, bei denen Targetmoleküle (z.B. Ionenkanäle, Rezeptoren, Enzyme, etc.) in eine auf einem sphärischen, festen Substrat immobilisierte Lipiddoppelschicht eingebracht werden. Die biochemische Aktivität dieser Targetmoleküle kann dann durch unterhalb der Lipiddoppeischicht befindliche Indikatorenoleküle nachgewiesen werden [Loidl-Stahlhofen et al. (2001) Solid-Supported ßiomolecules on Modified Silica Surfaces - A Tool for Fast Physicochemical Characterization and High-Throughput Screening. Advanced Materials 13, 1829-1834]. Das zumeist feste Substrat ist beispielsweise ein Silica-Träger, auf welchem eine Lipiddoppelschicht aus Lecitin aufgebracht ist. Eine solche Anordnung ist u.a. kommerziell erhältlich unter dem Handelsnamen Transil^{(R)} bei der Firma Nimbus Biotechnologie (Leipzig, Germany). Zum Nachweis der biochemischen Aktivität von Targetmolekülen (welche direkt in die Lipiddoppcischicht eingelagert sind) werden hierbei unterhalb der Lipiddoppclschicht immobilisierte Calcium-sensitive oder Phosphat-sensitive Farbstoffe verwendet.

Das vorstehend beschriebene Verfahren hat jedoch den Nachteil, dass für die Untersuchung eines jeden Targetmoleküls die entsprechend beschichteten und mit dem Targetmolekül angereicherten Substrate hergestellt werden müssen. Außerdem kann nicht immer gewährleistet werden, dass die Targetmoleküle in dieser "künstlichen" Umgebung die gleichen biochemischen Aktivitäten entfalten, wie in der "natürlichen" zellulären Umgebung.

### Connexine, Connexone und Gap Junctions

EP1660886A beschreibt Vorrichtungen und Verfahren zum Nachweis von biochemischer Aktivität mit einer Nachweisreaktion, wobei die nachzuweisende biochemische Aktivität räumlich getrennt von der Nachweisrcaktion verläuft. Die räumliche Trennung wird dadurch erreicht, dass Orte, in denen die zu untersuchende biochemische Aktivität bzw. die Nachweisreaktion stattfindet, durch mindestens zwei Lipidmembranen getrennt sind. Die Lipidmembranen sind jeweils mit Connexinen oder Innexinen besetzt, sodass sich zwischen den Membranen Gap Junctions (oder auch elektrische Synapse bezeichnet) ausbilden, die einen Durchtritt von Molekülen oder anderen Signalen zwischen dem Ort der biochemischen Aktivität und dem On der Nachweisreaktion ermöglichen.

Da Gap Junctions üblicherweise sowohl in die künstliche Lipidmembran als auch in die Lipidmembranen der Vesikel bzw. Zellen eingeführt werden müssen, ist die Herstellung des Membran-Konstrukts aus EP1660886A insbesondere für die Anwendung in High Throughput Screenings (HTS) sehr aufwändig.

### Fusion von Bilayer-Membranen

Die Fusion von zwei Bilayer-Membranen, die zur Ausbildung einer kontinuierlichen Membran führt, spielt bei vielen elementaren Lebensprozessen eine entscheidende Rolle. Als nur zwei Beispiele zu nennen wären die Exocytose, z.B. bei der Ausschüttung von Neurotransmittern an Synapsen, oder die Vorgänge bei der Infektion von Zellen durch bestimmte Viren. In den genannten Fällen wird die Membran-Fusion in der Regel durch komplizierte Interaktionen von Proteinen präzise gesteuert. Bei der Untersuchung von Membranproteinen in künstlichen planaren Lipidmembranen wird häufig die Fusion von Vesikeln mit der Membran induziert, indem ein osmolarer Gradient oder die Gegenwart von geladenen Ionen wie z.B. Calcium die Fusion herbeiführen soll. Dazu müssen aber spezielle Bedingungen ermittelt und eingehalten werden, so dass von einer breiten oder gar automatisierten Anwendung dieser Verfahren keine Rede sein kann. Die Fusion von Membranen kann auch durch eine Reihe von verschiedenen Substanzen herbeigeführt oder begünstigt werden, zu nennen wären z.B. bestimmte virale Proteine oder auch Antibiotika wie Polymyxin B. Bekannt ist auch, dass mit wasserlöslichen Polymeren wie z.B. Polyethylenglycol (PEG) eine Fusion von Lipidmembranen herbeigeführt werden kann.

### Beschreibung der Erfindung

Ausgehend von dem oben beschriebenen Stand der Technik stellt sich hier die technische Aufgabe, Mittel bereitzustellen, bei denen einerseits für zelluläre Assays nicht einsetzbare Indikatorsysteme (z.B. Reagenzien, die die Zellmembran nicht überwinden können oder elektrische Detektorsysteme, die einen Zugang zu beiden Seiten des von der Membran durchtrennten Raumes benötigen) angewendet werden können und andererseits die untersuchten Targetmoleküle in ihrer natürlichen zellulären Umgebung untersucht werden können, wobei diese Mittel einfach zugänglich sein sollten, um eine IITS-Anwendung zu ermöglichen.

Die technische Aufgabe wird erfindungsgemäß dadurch gelöst, dass eine Messanordnung bereitgestellt wird, bei der die nachzuweisende biochemische Aktivität innerhalb eines Membrankörpers und / oder die Membranaktivität des Membrankörpers durch Fusion seiner Membran mit einer Lipidmembran in einem Membran-Konstrukt zur Untersuchung zugänglich gemacht wird, wobei das Membran-Konstrukt durch Inkontaktbringen einer Lipidmembran mit mindestens einem Membrankörper und Fusion der Membranen mit Hilfe Membranfusion induzierender Stoffe herstellbar ist. Die erzeugten Membran-Konstrukte sind clektrophysiologisch oder biochemisch adressierbar. Die nachzuweisende biochemische Aktivität kann mittels mindestens eines Detektors registriert werden.

"Biochemische Aktivität", im Sinne der Erfindung, ist jedwede biochemische Aktivität, die in oder an der Oberfläche von biologischen Systemen (z.B. Zellen) stattfindet, stattfinden kann oder durch das biologische System katalysiert werden kann. Biochemische Aktivitäten sind z.B. durch Proteine katalysierte chemische Reaktionen, Signaltransduktionsvorgänge oder Änderungen der physikalischen oder chemischen Zustandsgrößen (wie z.B. pH-Wert, Ionenkonzentration, Metabolitkonzentrationen, etc.). Die Proteine, welche Targetmoteküle darstellen, können gelöst, z.B frei in Cytoplasma, oder auch inembrangebunden oder auch membranassoziiert sein, z.B. an der Cytoptasmamembran oder an Organellen, vorliegen.

Der erste Gegenstand der vorliegenden Erfindung betrifft eine Messanordnung zum Nachweis biochemischer Aktivität, wobei die nachzuweisende biochemische Aktivität innerhalb eines oder mehrerer Membrankörper und / oder die Membranaktivität der Membrankörper durch Fusion ihrer Membran mit mindestens einer Lipidmembran in einem oder mehreren Membran-Konstrukten zur Untersuchung zugänglich gemacht wird, wobei das Membran-Konstrukt durch Inkontaktbringen einer Lipidmembran mit mindestens einem Membrankörper und Fusion der Membranen mit Hilfe Membranfusion induzierender Stoffe herstellbar ist und mindestens einen, zu untersuchenden biochemisch aktiven Stoff enthält.

"Lipidmcmbranen", im Sinne der Erfindung, sind Lipidmembranen, wie sie dem Fachmann aus biologischen oder nicht-biologischen Systemen bekannt sind. Lipidmembranen enthalten bevorzugt eine Lipiddoppelschicht, welche den freien Durchtritt von hydrophilen Substanzen verhindert. In Lipidrnernbranen im Sinne der Erfindung können Moleküle, z.B. Proteine, eingelagert sein. Sie können planare so genannte suspended Bilayer oder planare oder sphärische so genannte supported Bilayer sein, insbesondere auch auf einer festen oder Gel-artigen Unterlage vorgesehen sein.

,,Suspended Bilayer" sind Membrane, die auf beiden Seiten von wässrigen Lösungen umgeben sind und die lateral durch einen Festköper begrenzt werden.

"Supported Bilayer" sind Membrane, die sich auf der einen Seite in Kontakt mit oder in unmittelbarer Nähe von einem geeigneten festen, porösen oder gel-artigen Material befinden. Dadurch werden sie gegenüber frei tragenden Membranen mechanisch stabiler und belastbarer.

"Membrankörper", im Sinne der Erfindung, sind von einer Membran umschlossene, mit einer Flüssigkeit gefüllte Volumenclemente. Erfindungsgemäße Membrankörper sind bevorzugt biologische Membrankörper, wie z.B. lebende Zellen. Dazu gehören Zelle, die aus lebendem Gewebe durch Dissoziation isoliert wurden (Primärkulturen). Dazu gehören ebenso Zellen, die als etablierte Zelllinien in Kultur gehalten werden, wie CHO-Zellen, HEK-Zellen, NIH3T3-Zellen, HeLa-Zellen aber auch transient oder stabil transfizierte Zellen oder Primärzellen. Biologische Membrankörper, im Sinne der Erfindung sind außerdem künstlich erzeugte Membrankörper, bei weichen z.B. eine Lipiddoppelschicht ein begrenztes Volumen eines wässrigen Mediums einschließt (Vesikel). Diese Membrankörper enthalten dann vorzugsweise mindestens eine biologische Komponente, z.B. ein in der Lipiddoppelschicht eingelagertes Polypeptid, ein membranständiges Enzym, einen lonenkanal oder einen G-Protein gekoppelten Rezeptor. Biologische Membrankörper, im Sinne der Erfindung können auch bakterielle Zellen, Pilzellen oder Zellen anderer einzelliger oder mehrzelliger Organismen sein. Biologische Membrankörper, im Sinne der Erfindung, sind z.B. auch Protoplasten von Pilzzellen und Pflanzenzellen, die durch Entfernen außenliegender Zellwände oder ähnlicher Strukturen erzielt werden. Biologische Membrankörper, im Sinne der Erfindung sind weiterhin auch Membrankörper, die - wie z.B. Synaptosomcn - durch Abspaltung oder Vereinigung aus den Membranen von lebenden Organismen erzeugt oder die durch die Vereinigung solcher Präparate mit synthetischen Lipidvesikeln erhalten worden sind.

Üblicherweise verfügt die erfindungsgemäße Messanordnung über mindestens einen Detektor für die elektrische Messung der biochemischen Aktivität und / oder einen Detektor zur Detektion einer biochemischen Nachweisreaktion.

"Detektoren" im Sinne der Erfindung sind Stromstärke-, Spannungs-, Widerstands- und Impedanz-Detektoren, Spektroskopische Detektoren wie z.B. Fluoreszenzdetektoren, Lumineszenzdetektoren, UV/VIS- Dciektoren, Tnfrarot-Detektoren, sowie Massenspektometrische Detektoren.

In einer ersten Ausführungsform der Erfindung wird die biochemische Aktivität durch Detektion einer Nachweisreaktion nachgewiesen.

"Nachweisreaktionen", im Sinne der Erfindung, sind chemische oder biochemische Reaktionen, welche die biochemische Aktivität oder deren Auswirkungen nachweisen können bzw. detektierbar machen. Bevorzugte Nachweisreaktionen sind Farbreaktionen, Fluoreszenz- oder Lumineszenzcrscheinungen oder auch komplexe biochemische Reaktionen, die den Nachweis der biochemischen Aktivität erlauben.

Am Ort der Nachweisreaktion wird die Änderung durch die Nachweisreaktion ggf. verstärkt bzw. detektierbar gemacht und schließlich detektiert. Die Detektion selbst kann irgendwo erfolgen. Die erfindungsgemäße Vorrichtung weist üblieherweise hierfür mindestens einen Detektor auf einer Seite des Membran-Konstrukts auf.

Zum Nachweis von biochemischer Aktivität mit einer Nachweisreaktion werden üblicherweise Indikatorsysteme (z.B. Reagenzien, die die Zellmembran nicht überwinden können) verwendet. Indikatorsysteme sind beispielsweise Farbstoffe, radioaktive Markierungen, Calcium-sensitive Indikatoren, Spannungs-sensitive Indikatoren oder pH-Wert-sensitive Indikatoren.

"Farbstoffe", im Sinne der Erfindung, sind Stoffe, die optisch durch Detektion der von ihnen ausgesandten oder der durch sie absorbierten oder nicht absorbierten elektromagnetischen Strahlung nachgewiesen werden können.

"Radioaktive Markierungen", im Sinne der Erfindung sind radioaktive Substanzen, die bei radioaktivem Zerfall ionisierende Strahlung aussenden, die nachgewiesen werden kann.

"Spannungs-sensitive Indikatoren", im Sinne der Erfindung, sind Stoffe, die in Abhängigkeit einer anliegenden elektrischen Potentialdifferenz oder des vorliegenden elektrischen Potentials derart ihre physikalischen, optischen oder katalytischen Eigenschaften ändern, dass diese ein detektierbares Signal hervorrufen.

Dem Fachmann bekannt sind spannungs-sensitive Indikatoren wie z.B. DIBAC.

"pH-Wert-sensitive Indikatoren", im Sinne der Erfindung, sind Stoffe, die in Abhängigkeit des pH-Wertes derart ihre physikalischen, optischen oder katalytischen Eigenschaften ändern, dass diese ein detektierbares Signal hervorrufen. Solche Indikatorfarbstoffe, wie z. B. Phenolrot, ßromthymolblau, Bromphenolblau u.v.a. sind dem Fachmann zahlreich bekannt.

"Caicium-sensitive Indikatoren", im Sinne der Erfindung, sind Stoffe, die in Anwesenheit von Calcium derart ihre physikalischen, optischen oder katalytischen Eigenschaften ändern, dass diese ein detektierbares Signal hervorrufen. Dem Fachmann bekannte Calcium-sensitive Indikatoren sind z.B. Aequorin und andere Calcium-sensitive Farbstoffe wie z.B. FURA-2.

In einer weiteren Ausführungsform der Erfindung wird die biochemische Aktivität elektrisch bemessen. Hierfür werden bevorzugt ein oder mehrere Stromstärken- bzw. Spannungsdetektoren verwendet. In einer bevorzugten Ausführungsform der erfindungsgemäßen Vorrichtung befindet sich jeweils eine Elektrode in den durch das Membran-Konstrukt getrennten Räumen.

Ein weiterer Gegenstand der vorliegenden Erfindung ist die Verwendung der erfindungsmäßen Messvorrichtung zum Screening bzw. High Throughput Screening von Wirkstoffen.

"Wirkstoffe", im Sinne der Erfindung, sind Stoffe, welche die Aktivität von biologischen Molekülen beeinflussen können. Bevorzugte Wirkstoffe, im Sinne der Erfindung, sind solche, die spezifisch die Aktivität von einzelnen biologischen Molekülen oder von Gruppen von biologischen Molekülen beeinflussen. Besonders bevorzugte Wirkstoffe sind solche, welche die Aktivität von Rezeptoren und/oder Ionenkanälen beeinflussen. Ganz besonders bevorzugte Wirkstoffe vermögen bestimmte Krankheitsbilder des Menschen oder von Tieren zu heilen, zu lindern oder zu verhindern.

"Wirkstoff-Screening", in Sinne der Erfindung, ist die zielgericlitete Suche nach chemischen oder biologischen Substanzen, die bei einem bestimmten biologischen System einen bestimmten physiotogischen Effekt auslösen. Dieser Effekt ist bevorzugt die Modulation der Aktivität eines Targetmoleküls oder die Heilung, Linderung, oder Verhinderung eines bestimmten Krankheitszustands eines Organismus. Wirkstoff-Screening wird bevorzugt im High-Throughput-Screening (HTS) Format durchgeführt. Hierbei wird eine große Anzahl von chemischen Substanzen während des Screening-Verfahrens mit einem Target in Kontakt gebracht und die Auswirkungen der chemischen Substanzen auf das Target wird ausgewertet.

Ein weiterer Gegenstand der vorliegenden Erfindung ist ein Verfahren zur Ilerstellung einer Messanordnung enthaltend mindestens ein Membran-Konstrukt, wobei mindestens eine Lipidmembran mit mindestens einem Membrankörper in Kontakt gebracht wird und die Membranen mit Hilfe Membranfusion induzierender Stoffe zur Herstellung des Membran-Konstrukts fusioniert worden.

Typischerweise werden als Lipidmembran, planare suspended Bilayer oder planare oder sphärische supported Bilayer verwendet, die durch bekannten Verfahren vorbereitet werden können. Zur Herstellung eines planaren Bilayer wird beispielsweise die Methode von H. Ticn. [H. Tien., Journal of Membrane Science, 189(1):83-117, July 2001]. Suspended Bilayer können z. B durch einer der von M. Trojanowicz in Fresenius' journal of analytical chemistry, 371(2):246-260, September 2001 beschriebene Methode bereitgestellt werden. Zur Ilerstellung von supported Bilayer sind viele Methode bekannt, die in folgenden Übersichtartikel aufgeführt sind [M. Tanaka, E. Sackmann, Nature, 437(7059):656-663, September 2005 ; E. Sackmann and M. Tanaka, Trends in biotechnology, 18(2):58-64, February 2000; Ingo Kooper, Molecular bioSystems, 3(10):651-657, October2007].

Vorzugsweise werden als Lipidmembran
- ein planarer supported, oder suspcndod Bilayer auf einem Träger, oder
- ein geschlossener supported Bilayer an der Oberfläche eines partikulärem Trägers oder
- ein geschlossener suspended Bilayer auf einem porösen partikulären Substrat, oder
- ein oder mehrere mit Flüssigkeit oder Gel gefüllte Vesikel verwendet.

Besonders bevorzugt werden planare supported oder suspended Bilayer, besonders bevorzugt planare supported Bilayer verwendet, da diese eine hohe Reproduzierbarkeit in ihrer Ilerstellung aufweisen und eine hohe mechanische Stabilität aufweisen.

Da die Membranfusion statistisch erfolgt, ist es vorteilhaft von 10 bis 10.000 Zellen bzw. Vesikel, bevorzugt 50 bis 5000 mit der Lipidmembran in Kontakt zu bringen. Bei dem Herstellungsverfahren findet ebenfalls eine Fusion zwischen Membrankörpern statt, die jedoch nicht detektiert werden, Insbesondere bietet eine planare Lipidmembran die größte Kontaktfläche mit den Membrankörpern, entsprechend steigt die Wahrscheinlichkeit, dass eine Fusion zwischen der planaren Lipidmembran und der Membrankörpermembran stattfinden wird.

In einer besonderen Ausführungsform des erfindungsgemäßen Verfahrens ist die Lipidmembran planar, Hierbei sind die Membrankörper bevorzugt kleiner als die Lipidmembranen, so dass mehr als ein Membrankörper mit der Lipidmembran fusionierett kann.

Geeignete Membranfusion induzierende Stoffe unterteilen sich in drei Klassen:
a) Stoffe der Klasse A, die eine Fusion von Membranen hervorrufen können, ohne dass diese in die Membran selbst integriert sein müssen.
b) Stoffe der Klasse B, die eine Fusion von Membranen hervorrufen können, dafür sich jedoch in die Membran einlagern müssen. Diese sind teilweise bereits in elektrophysiologischen Messungen eingesetzt worden, um den Ablauf der Fusion zu charakterisieren, jedoch nicht um die fusionierte Zelle zu charakterisieren [Bsp. G. B. Melikyan, R. M. Markosyan, S. A. Brcncr, Y. Rozenberg, F. S. Cohen, J. Virol. 74, 447 (2000) ; Chernomordik LV, Kozlov MM, 2008, Mechanics of membrane fusion, Nature Strucrural & Molecular Biology, 5,7, 675-683].
c) Stoffe der Klasse C sind fusogene Proteine oder Peptide [Übersichtartikel: Chernomordik LV, Kozlov MM, 2008, Nature Structural & Molecular Biology, 5,7, 675-683 ; Amir Sapir, Ori Avinoam, Benjamin Podbilewicz, and Leonid V. Chernomordik, Developmental cell, 14(1):11-21, January 2008]

Ein typisches Beispiel für Membranfusion induzierende Stoffe der Klasse A ist Ethylenglycol bzw. Polyethylenglycol [Robinson JM, Roos DS, Davidson RL, Karnovsky MJ, J.Cell Sei 40, 63-75 (1979); de StGroth SF, Scheidegger D, J Immunol Methods. 1980;35(1-2):1-21; S. W. Hui, T. Isac, L. T. Boni, and A. Sen., 84(2):137-146, June 1985].

Beispiele für Membranfusion induzierende Stoffe der Klasse B sind Phosphatidylethanolamine, Diacylglycerol, Phosphatidylserine, D-Ala-D-Ala [Gong Y, Luo Y, and Bong D (2006) Membrane Activation: Selective Vesicle Fusion via Small Molecule Recognition J. Am. Chem. Soe., 128 (45), pp 14430-14431]
Beispiele für fusogene Proteine oder Peptide (Klasse C) sind S-glycoprotein (SARS Virus) [Simmons, G., Gosalia, D.N., Rennekamp, A.J., Reeves, J.D., Diamond, S.L., and Bates, P. (2005), Proe. Natl. Acad. Sci. USA 102, 11876-11881], Influenza Hemaglutinin Protein und davon abgeleitete Peptide [D V Zhelev, N Stoicheva, P Scherrer, and D Needham (2001), Biophys J. 81(1), 285-304]

Membranfusion induzierende Stoffe der Klasse A werden bevorzugt verwendet. Besonders bevorzugt wird Polyethylenglycol verwendet, das typischerweise mit einer Konzentration von 10 bis 50 Vol% und die in den Veröffentlichungen angegebenen Konzentrationen hinzugefügt wird.

Üblicherweise wird mindestens ein Detektor auf einer Seite des Membran-Konstrukts eingesetzt. Bevorzugt werden ein Stromstärke- und Spannungsdetektor verwendet. In einer bevorzugten Ausführungsform der erfindungsgemäßen Verfahren wird jeweils eine Elektrode in den durch das Metnbran-Konstrukt getrennten Räumen eingesetzt.

Ein weiterer Gegenstand der vorliegenden Erfindung ist die Anwendung von Membranfusion induzierenden Stoffen zur Herstellung einer Messanordnung zum Nachweis von biocheinischer Aktivität, wobei die nachzuweisende biochemische Aktivität innerhalb von Membrankörper und / oder die Membranaktivität der Membrankörper durch Fusion seiner Membran mit einer Lipidmembran in einem Membran-Konstrukt zur Untersuchung zugänglich gemacht wird.

### Besehreibung der Abbildungen

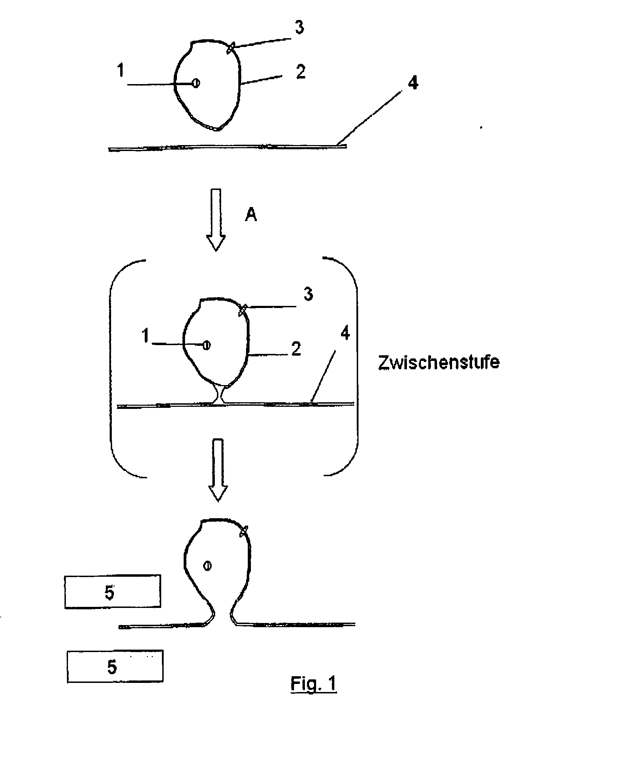

### Figur 1: graphische Darstellung der Herstellung des Membran-Konstrukts.

### ßezugszeichen

1 in wässriger Phase gelöster Stoff. Der Zugang zu diesem Stoff ist durch die Membranhülle beschränkt
2 Membran Vesikel oder Zelle
3 Membrangcbundener Stoff z.B. Protein mit biologischer Funktion
4 Planacer Lipid Bilayer oder Membran
5 Detektor
A Fusogene Stoffe

## Patentansprüche

1. Messanordnung zum Nachweis von biochemischer Aktivität, wobei die nachzuweisende biochemische Aktivität innerhalb eines oder mehrerer Membrankörper und / oder die Membranaktivität der Membrankörper durch Fusion ihrer Membranen mit mindestens einer Lipidmembran in einem oder mehreren Membran-Konstrukten zur Untersuchung zugänglich gemacht wird, wobei das Membran-Konstrukt durch Inkontaktbringen einer Lipidmembran mit mindestens einem Membrankörper und Fusion der Membranen mit Hilfe Membranfusion induzierender Stoffe herstellbar ist und mindestens einen, zu untersuchenden, biochemisch aktiven Stoff enthält.

2. Messanordnung gemäß Anspruch 1, wobei die Lipidmembran eine planare Lipidmembran oder eine geschlossene Lipidmembran ist.

3. Messanordnung gemäß Anspruch 2, wobei die planare Lipidmembran ein supported oder ein suspended Bilayer ist.

4. Messanordnung gemäß Anspruch 2, wobei die geschlossene Lipidmembrane ein supported Bilayer auf einem partikulären Träger, ein suspended Bilayer auf einem porösen Partikel oder ein mit Flüssigkeit oder Gel gefülltes Vesikel ist.

5. Messanordnung, nach einem der Ansprüche 1 bis 5, die mindestens einen Detektor für elektrische Messung der biochemischen Aktivität und / oder einen Detektor zur Detektion einer biochemischen Nachweisreaktion aufweist.

6. Verwendung der Messanordnung nach einem der Ansprüche 1 bis 5 zum Screening bzw. High Throughput Scrcening von Wirkstoffen.

7. Verfahren zur Herstellung einer Messanordnung enthaltend mindestens ein Membran-Konstrukt, bei dem mindestens ein Membrankörper mit mindestens einer Lipidmembran in Kontakt gebracht wird, und die Fusion des Membrankörpers mit der Lipidmembran durch Membranfusions-induzierende Stoffe herbeigeführt wird.

8. Verfahren nach Anspruch 7, wobei die Lipidmembran
a. ein planarer supported oder planarer suspended Bilayer auf einem Träger, oder
b. ein geschlossener supported Bilayer an der Oberfläche eines partikulären Trägers oder
c. ein geschlossener suspended Bilayer auf einem porösen partikulären Substrat, oder
d. ein oder mehrere mit Flüssigkeit oder Gel Gefülltes Vesikel ist.

9. Verfahren nach Anspruch 8, **gekennzeichnet dadurch, dass** die Lipidmcmhran ein planarer supported oder ein planarer suspended Bilayer auf einem Träger ist und die Membrankörper kleiner als die Lipidmembran ist, so dass mehr als ein Membrankörper mit der Lipidmembran fusionieren kann.

10. Verfahren nach einem der Ansprüche 7 bis 9, wobei Membranfusion induzierende Stoffe solche Stoffe sind, die eine Fusion von Membranen hervorrufen können, ohne dass diese in die Membran selbst integriert sein müssen.

11. Verfahren nach einem der Ansprüche 7 bis 10, wobei der Membranfusion induzierende Stoff Ethylenglycol oder Polyethylcnglycol ist.

12. Verwendung von Membranfusion induzierenden Stoffen zur Herstellung einer Messanordnung zum Nachweis von biochemischer Aktivität mit einer Nachweisreaktion und / oder elektrischen Messung, wobei die nachzuweisende biochemische Aktivität innerhalb der Membrankörper und / oder die Membranaktivität der Membrankörper durch Fusion ihrer Membran mit einer Lipidmembran in einem Membran-Konstrukt zur Untersuchung zugänglich gemacht wird.
